# EUROPEAN PATENT SPECIFICATION

(11) **EP 2 729 004 B1**
(45) Date of publication and mention of the grant of the patent: **25.03.2020**
(21) Application number: 12807735.1
(22) Date of filing: 05.07.2012
(51) Int. Cl.: A61K 31/12, A01N 35/00, A61P 3/00

(54) **TREATMENT OF METHYLMALONIC ACIDURIA, ISOVALERIC ACIDURIA, AND OTHER ORGANIC ACIDURIAS WITH TOCOTRIENOL QUINONES**
BEHANDLUNG VON METHYLMALONAZIDURIE, ISOVALERIANSÄUREAZIDURIE UND ANDEREN ORGANISCHEN AZIDURIEN MIT TOCOTRIENOLCHINONEN
TRAITEMENT D'UNE ACIDURIE MÉTHYLMALONIQUE, D'UNE ACIDURIE ISOVALÉRIQUE ET D'AUTRES ACIDURIES ORGANIQUES PAR DES TOCOTRIÉNOL QUINONES

(30) Priority: 06.07.2011 US 201161505051 P
(43) Date of publication of application: 14.05.2014
(73) Proprietor: PTC Therapeutics, Inc., South Plainfield, NJ 07080 (US)
(72) Inventor: MILLER, Guy, M., Mountain View, CA 94043 (US); DIONISI-VICI, Carlo, I-1-00165 Rome (IT); BERTINI, Enrico, I-1-00165 Rome (IT); MARTINELLI, Diego, I-1-00165 Rome (IT)
(74) Representative: J A Kemp LLP
(86) International application number: PCT/US2012/045628
(87) International publication number: WO 2013/006737

(56) References cited:
- WO-A1-2011/072281
- WO-A1-2011/072281
- US-A1- 2008 187 911
- US-A1- 2010 273 892
- OKUN ET AL.: 'Neurodegeneration in Methylmalonic Aciduria Involves Inhibition of Complex II and the Tricarboxylic Acid Cycle, and Synergistically Acting Excitotoxicity' JOURNAL OF BIOLOGICAL CHEMISTRY vol. 277, no. 17, 26 April 2002, pages 14674 - 14680, XP055139212
- SHRADER ET AL.: 'Alpha-Tocotrienol quinone modulates oxidative stress response and the biochemistry of aging.' BIOORGANIC & MEDICINAL CHEMISTRY LETTERS. vol. 21, no. 12, June 2011, pages 3693 - 8, XP002663593
- , Retrieved from the Internet: URL:http://www.hmdb.ca/metabolites/HMDB006 91 [retrieved on 2017-02-28]
- , 7 January 2016 (2016-01-07), Retrieved from the Internet: URL:www.ncbi.nlm.nih.gov/books/NBK1231
- , 21 October 2015 (2015-10-21), Retrieved from the Internet: URL:www.omim.org/entry/252011 [retrieved on 2017-02-28]

## Description

### TECHNICAL FIELD OF THE INVENTION

The present invention relates to compounds for use in a method of treating methylmalonic aciduria, isovaleric aciduria, and other organic acidurias with tocotrienol quinones (including tocotrienol hydroquinones), for example, alpha-tocotrienol quinone.

### BACKGROUND OF THE INVENTION

Organic acidurias (also known as organic acidemias) are a set of disorders caused by defects in amino acid metabolism, resulting in an abnormal accumulation of acids in the body. See Rezvani I., "Defects in metabolism of amino acids," in: Kliegman RM, Behrman RE, Jenson HB, Stanton BF, eds. Nelson Textbook of Pediatrics. 18th ed. Philadelphia, Pa: Saunders Elsevier; 2007, Chap 85.

Methylmalonic aciduria (methylmalonic acidemia, MMA) is caused by defects in methylmalonyl-CoA mutase or its cofactor, cobalamin (vitamin B12). MMA is accompanied by progressive encephalopathy, dehydration, feeding problems, developmental delays, failure to thrive, lethargy, recurrent yeast infections, seizures, emesis, and can result in kidney disease, kidney failure, pancreatitis, coma, or death. See World-Wide-Web.ncbi.nlm.nih.gov/pubmedhealth/PMH0002147/ and URL ghr.nlm.nih.gov/condition/methylmalonic-acidemia. Brain abnormalities demonstrated in MMA include ventricular dilation, cortical atrophy, periventricular white matter abnormality, thinning of the corpus callosum, subcortical white matter abnormality, cerebellar atrophy, basal ganglionic calcification, and myelination delay (see Radmanesh et al., Pediatr Radiol (2008) 38:1054-1061).

Another organic aciduria, isovaleric acidemia (isovaleric aciduria; IVA) is caused by a deficiency in the activity of isovaleryl-CoA dehydrogenase which catalyzes the metabolism of leucine resulting in an accumulation of isovaleric acid. The features of isovaleric acidemia become apparent within a few days after birth and include poor feeding, vomiting, seizures and lack of energy. The symptoms can progress to seizures, coma and possibly death. In some cases the symptoms appear during childhood, where children fail to gain weight and fail to thrive. A characteristic sign of isovaleric acidemia is a distinctive odor of sweaty feet during acute illness.

Control of organic acidurias often relies on strict adherence to specific diets lacking branched-chain amino acids.

There is thus a need for effective treatments for methylmalonic aciduria, isovaleric aciduria, and other organic acidurias.

### SUMMARY OF THE INVENTION

The invention provides a compound selected from the group consisting of tocotrienol quinones and tocotrienol hydroquinones for use in a method of treating methylmalonic aciduria, isovaleric aciduria, or another organic aciduria in an individual.

In one embodiment, the compounds for use in a method of treating an individual suffering from methylmalonic aciduria, isovaleric aciduria, or another organic aciduria are tocotrienol quinones, and the method comprises administering a therapeutically effective amount of one or more tocotrienol quinones to an individual suffering from methylmalonic aciduria, isovaleric aciduria, or another organic aciduria. In another embodiment, the compound for use in a method of treating an individual suffering from methylmalonic aciduria is alpha-tocotrienol quinone, and the method comprises administering a therapeutically effective amount of alpha-tocotrienol quinone to an individual suffering from methylmalonic aciduria. In another embodiment, the compound for use in a method of treating an individual suffering from isovaleric aciduria or another organic aciduria is alpha-tocotrienol quinone, and the method comprises administering a therapeutically effective amount of alpha-tocotrienol quinone to an individual suffering from isovaleric aciduria or another organic aciduria. In another embodiment, the compound for use in a method of treating an individual suffering from methylmalonic aciduria is beta-tocotrienol quinone, and the method comprises administering a therapeutically effective amount of beta-tocotrienol quinone to an individual suffering from methylmalonic aciduria. In another embodiment, the compound for use in a method of treating an individual suffering from isovaleric aciduria or another organic aciduria is beta-tocotrienol quinone, and the method comprises administering a therapeutically effective amount of beta-tocotrienol quinone to an individual suffering from isovaleric aciduria or another organic aciduria. In another embodiment, the compound for use in a method of treating an individual suffering from methylmalonic aciduria is gamma-tocotrienol quinone, and the method comprises administering a therapeutically effective amount of gamma-tocotrienol quinone to an individual suffering from methylmalonic aciduria. In another embodiment, the compound for use in a method of treating an individual suffering from isovaleric aciduria or another organic aciduria is gamma-tocotrienol quinone, and the method comprises administering a therapeutically effective amount of gamma-tocotrienol quinone to an individual suffering from isovaleric aciduria or another organic aciduria. In another embodiment, the compound for use in a method of treating an individual suffering from methylmalonic aciduria is delta-tocotrienol quinone, and the method comprises administering a therapeutically effective amount of delta-tocotrienol quinone to an individual suffering from methylmalonic aciduria. In another embodiment, the compound for use in a method of treating an individual suffering from isovaleric aciduria or another organic aciduria is delta-tocotrienol quinone, and the method comprises administering a therapeutically effective amount of delta-tocotrienol quinone to an individual suffering from isovaleric aciduria or another organic aciduria.

In another embodiment, the compounds for use in a method of treating an individual suffering from methylmalonic aciduria, isovaleric aciduria, or another organic aciduria are tocotrienol hydroquinones, and the method comprises administering a therapeutically effective amount of one or more tocotrienol hydroquinones to an individual suffering from methylmalonic aciduria, isovaleric aciduria, or another organic aciduria. In another embodiment, the compound for use in a method of treating an individual suffering from methylmalonic aciduria is alpha-tocotrienol hydroquinone, and the method comprises administering a therapeutically effective amount of alpha-tocotrienol hydroquinone to an individual suffering from methylmalonic aciduria. In another embodiment, the compound for use in a method of treating an individual suffering from isovaleric aciduria or another organic aciduria is alpha-tocotrienol hydroquinone, and the method comprises administering a therapeutically effective amount of alpha-tocotrienol hydroquinone to an individual suffering from isovaleric aciduria or another organic aciduria. In another embodiment, the compound for use in a method of treating an individual suffering from methylmalonic aciduria is beta-tocotrienol hydroquinone, and the method comprises administering a therapeutically effective amount of beta-tocotrienol hydroquinone to an individual suffering from methylmalonic aciduria. In another embodiment, the compound for use in a method of treating an individual suffering from isovaleric aciduria or another organic aciduria is beta-tocotrienol hydroquinone, and the method comprises administering a therapeutically effective amount of beta-tocotrienol hydroquinone to an individual suffering from isovaleric aciduria or another organic aciduria. In another embodiment, the compound for use in a method of treating an individual suffering from methylmalonic aciduria is gamma-tocotrienol hydroquinone, and the method comprises administering a therapeutically effective amount of gamma-tocotrienol hydroquinone to an individual suffering from methylmalonic aciduria. In another embodiment, the compound for use in a method of treating an individual suffering from isovaleric aciduria or another organic aciduria is gamma-tocotrienol hydroquinone, and the method comprises administering a therapeutically effective amount of gamma-tocotrienol hydroquinone to an individual suffering from isovaleric aciduria or another organic aciduria. In another embodiment, the compound for use in a method of treating an individual suffering from methylmalonic aciduria is delta-tocotrienol hydroquinone, and the method comprises administering a therapeutically effective amount of delta-tocotrienol hydroquinone to an individual suffering from methylmalonic aciduria. In another embodiment, the compound for use in a method of treating an individual suffering from isovaleric aciduria or another organic aciduria is delta-tocotrienol hydroquinone, and the method comprises administering a therapeutically effective amount of delta-tocotrienol hydroquinone to an individual suffering from isovaleric aciduria or another organic aciduria.

In one embodiment, the pharmaceutical composition used in treating the individual comprises alpha-tocotrienol quinone, where the alpha-tocotrienol quinone comprises at least about 30% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 40% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 50% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 60% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 70% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 75% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 80% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 90% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 95% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 98% by weight of the tocotrienols and tocotrienol quinones present in the preparation. In another embodiment, the pharmaceutical composition used in treating the individual comprises alpha-tocotrienol quinone, where the alpha-tocotrienol quinone comprises at least about 99% by weight of the tocotrienols and tocotrienol quinones present in the preparation.

In one embodiment, the pharmaceutical composition used in treating the individual comprises alpha-tocotrienol quinone, where the alpha-tocotrienol quinone comprises at least about 30% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 40% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 50% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 60% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 70% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 75% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 80% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 90% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 95% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 98% by weight of the material present in the preparation, excluding the weight of any added pharmaceutical carriers or excipients. In another embodiment, the pharmaceutical composition used in treating the individual comprises alpha-tocotrienol quinone, where the alpha-tocotrienol quinone comprises at least about 99% by weight of the material present in the preparation, excluding the weight of any added pharmaceutical carriers or excipients.

Unit dosage formulations of between about 50 mg to 500 mg of alpha-tocotrienol quinone may be provided, where the purity of the alpha-tocotrienol quinone present in the formulation comprises at least about 30%, at least about 40%, at least about 50%, at least about 60%, at least about 70%, at least about 75%, at least about 80%, at least about 90%, at least about 95%, at least about 98%, or at least about 99% by weight of the tocotrienols and tocotrienol quinones present in the preparation. The unit dosage formulations can be used to treat an individual suffering from methylmalonic aciduria, isovaleric aciduria, or another organic aciduria.

Unit dosage formulations of between about 50 mg to 500 mg of alpha-tocotrienol quinone may be provided, where the purity of the alpha-tocotrienol quinone present in the formulation comprises at least about 30%, at least about 40%, at least about 50%, at least about 60%, at least about 70%, at least about 75%, at least about 80%, at least about 90%, at least about 95%, at least about 98%, or at least about 99% of the material present in the preparation, excluding the weight of any added pharmaceutical carriers or excipients. The unit dosage formulations can be used to treat an individual suffering from methylmalonic aciduria, isovaleric aciduria, or another organic aciduria.

Any of the pharmaceutical compositions, pharmaceutical formulations and unit dosage formulations of alpha-tocotrienol quinone can be used to treat an individual suffering from methylmalonic aciduria, isovaleric aciduria, or another organic aciduria, such as an individual with methylmalonic aciduria, such as an individual with methylmalonic aciduria where the individual has a mutation, one or more mutations, or two or more mutations in the MUT, MMAA, and/or MMAB gene, or an individual with isovaleric academia with a mutation, one or more mutations, or two or more mutations in the IVD gene.

In one embodiment, the pharmaceutical composition used in treating the individual comprises beta-tocotrienol quinone, where the beta-tocotrienol quinone comprises at least about 30% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 40% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 50% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 60% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 70% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 75% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 80% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 90% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 95% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 98% by weight of the tocotrienols and tocotrienol quinones present in the preparation. In another embodiment, the pharmaceutical composition used in treating the individual comprises beta-tocotrienol quinone, where the beta-tocotrienol quinone comprises at least about 99% by weight of the tocotrienols and tocotrienol quinones present in the preparation.

In one embodiment, the pharmaceutical composition used in treating the individual comprises beta-tocotrienol quinone, where the beta-tocotrienol quinone comprises at least about 30% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 40% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 50% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 60% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 70% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 75% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 80% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 90% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 95% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 98% by weight of the material present in the preparation, excluding the weight of any added pharmaceutical carriers or excipients. In another embodiment, the pharmaceutical composition used in treating the individual comprises beta-tocotrienol quinone, where the beta-tocotrienol quinone comprises at least about 99% by weight of the material present in the preparation, excluding the weight of any added pharmaceutical carriers or excipients.

Unit dosage formulations of between about 50 mg to 500 mg of beta-tocotrienol quinone may be provided, where the purity of the beta-tocotrienol quinone present in the formulation comprises at least about 30%, at least about 40%, at least about 50%, at least about 60%, at least about 70%, at least about 75%, at least about 80%, at least about 90%, at least about 95%, at least about 98%, or at least about 99% by weight of the tocotrienols and tocotrienol quinones present in the preparation. The unit dosage formulations can be used to treat an individual suffering from methylmalonic aciduria, isovaleric aciduria, or another organic aciduria.

Unit dosage formulations of between about 50 mg to 500 mg of beta-tocotrienol quinone may be provided, where the purity of the beta-tocotrienol quinone present in the formulation comprises at least about 30%, at least about 40%, at least about 50%, at least about 60%, at least about 70%, at least about 75%, at least about 80%, at least about 90%, at least about 95%, at least about 98%, or at least about 99% of the material present in the preparation, excluding the weight of any added pharmaceutical carriers or excipients. The unit dosage formulations can be used to treat an individual suffering from methylmalonic aciduria, isovaleric aciduria, or another organic aciduria.

Any of the pharmaceutical compositions, pharmaceutical formulations and unit dosage formulations of beta-tocotrienol quinone can be used to treat an individual suffering from methylmalonic aciduria, isovaleric aciduria, or another organic aciduria, such as an individual with methylmalonic aciduria, such as an individual with methylmalonic aciduria where the individual has a mutation, one or more mutations, or two or more mutations in the MUT, MMAA, and/or MMAB gene, or an individual with isovaleric aciduria with a mutation in the IVD gene.

In one embodiment, the pharmaceutical composition used in treating the individual comprises gamma-tocotrienol quinone, where the gamma-tocotrienol quinone comprises at least about 30% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 40% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 50% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 60% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 70% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 75% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 80% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 90% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 95% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 98% by weight of the tocotrienols and tocotrienol quinones present in the preparation. In another embodiment, the pharmaceutical composition used in treating the individual comprises gamma-tocotrienol quinone, where the gamma-tocotrienol quinone comprises at least about 99% by weight of the tocotrienols and tocotrienol quinones present in the preparation.

In one embodiment, the pharmaceutical composition used in treating the individual comprises gamma-tocotrienol quinone, where the gamma-tocotrienol quinone comprises at least about 30% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 40% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 50% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 60% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 70% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 75% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 80% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 90% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 95% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 98% by weight of the material present in the preparation, excluding the weight of any added pharmaceutical carriers or excipients. In another embodiment, the pharmaceutical composition used in treating the individual comprises gamma-tocotrienol quinone, where the gamma-tocotrienol quinone comprises at least about 99% by weight of the material present in the preparation, excluding the weight of any added pharmaceutical carriers or excipients.

Unit dosage formulations of between about 50 mg to 500 mg of gamma-tocotrienol quinone may be provided, where the purity of the gamma-tocotrienol quinone present in the formulation comprises at least about 30%, at least about 40%, at least about 50%, at least about 60%, at least about 70%, at least about 75%, at least about 80%, at least about 90%, at least about 95%, at least about 98%, or at least about 99% by weight of the tocotrienols and tocotrienol quinones present in the preparation. The unit dosage formulations can be used to treat an individual suffering from methylmalonic aciduria, isovaleric aciduria, or another organic aciduria.

Unit dosage formulations of between about 50 mg to 500 mg of gamma-tocotrienol quinone may be provided, where the purity of the gamma-tocotrienol quinone present in the formulation comprises at least about 30%, at least about 40%, at least about 50%, at least about 60%, at least about 70%, at least about 75%, at least about 80%, at least about 90%, at least about 95%, at least about 98%, or at least about 99% of the material present in the preparation, excluding the weight of any added pharmaceutical carriers or excipients. The unit dosage formulations can be used to treat an individual suffering from methylmalonic aciduria, isovaleric aciduria, or another organic aciduria.

Any of the pharmaceutical compositions, pharmaceutical formulations and unit dosage formulations of gamma-tocotrienol quinone can be used to treat an individual suffering from methylmalonic aciduria, isovaleric aciduria, or another organic aciduria, such as an individual with methylmalonic aciduria, such as an individual with methylmalonic aciduria where the individual has a mutation, one or more mutations, or two or more mutations in the MUT, MMAA, and/or MMAB gene, or an individual with isovaleric aciduria where the individual has a mutation, one or more mutations, or two or more mutations in the IVD gene.

In one embodiment, the pharmaceutical composition used in treating the individual comprises delta-tocotrienol quinone, where the delta-tocotrienol quinone comprises at least about 30% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 40% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 50% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 60% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 70% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 75% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 80% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 90% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 95% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 98% by weight of the tocotrienols and tocotrienol quinones present in the preparation. In another embodiment, the pharmaceutical composition used in treating the individual comprises delta-tocotrienol quinone, where the delta-tocotrienol quinone comprises at least about 99% by weight of the tocotrienols and tocotrienol quinones present in the preparation.

In one embodiment, the pharmaceutical composition used in treating the individual comprises delta-tocotrienol quinone, where the delta-tocotrienol quinone comprises at least about 30% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 40% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 50% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 60% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 70% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 75% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 80% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 90% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 95% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 98% by weight of the material present in the preparation, excluding the weight of any added pharmaceutical carriers or excipients. In another embodiment, the pharmaceutical composition used in treating the individual comprises delta-tocotrienol quinone, where the delta-tocotrienol quinone comprises at least about 99% by weight of the material present in the preparation, excluding the weight of any added pharmaceutical carriers or excipients.

Unit dosage formulations of between about 50 mg to 500 mg of delta-tocotrienol quinone may be provided, where the purity of the delta-tocotrienol quinone present in the formulation comprises at least about 30%, at least about 40%, at least about 50%, at least about 60%, at least about 70%, at least about 75%, at least about 80%, at least about 90%, at least about 95%, at least about 98%, or at least about 99% by weight of the tocotrienols and tocotrienol quinones present in the preparation. The unit dosage formulations can be used to treat an individual suffering from methylmalonic aciduria, isovaleric aciduria, or another organic aciduria.

Unit dosage formulations of between about 50 mg to 500 mg of delta-tocotrienol quinone may be provided, where the purity of the delta-tocotrienol quinone present in the formulation comprises at least about 30%, at least about 40%, at least about 50%, at least about 60%, at least about 70%, at least about 75%, at least about 80%, at least about 90%, at least about 95%, at least about 98%, or at least about 99% of the material present in the preparation, excluding the weight of any added pharmaceutical carriers or excipients. The unit dosage formulations can be used to treat an individual suffering from methylmalonic aciduria, isovaleric aciduria, or another organic aciduria.

Any of the pharmaceutical compositions, pharmaceutical formulations and unit dosage formulations of delta-tocotrienol quinone can be used to treat an individual suffering from methylmalonic aciduria, isovaleric aciduria, or another organic aciduria, such as an individual with methylmalonic aciduria, such as an individual with methylmalonic aciduria where the individual has a mutation, one or more mutations, or two or more mutations in the MUT, MMAA, and/or MMAB gene, or an individual with isovaleric aciduria where the individual has a mutation, one or more mutations, or two or more mutations in the IVD gene.

In one embodiment, the individual suffering from methylmalonic aciduria has a mutation, or at least one mutation, or two or more mutations, in a gene, or at least one gene, or two or more genes, selected from the group consisting of MUT, MMAA, and/or MMAB. In another embodiment, the individual suffering from methylmalonic aciduria has a mutation, or has at least one mutation, or has two or more mutations, in the MUT gene. In another embodiment, the individual suffering from methylmalonic aciduria has a mutation, or has at least one mutation, or has two or more mutations, in the MMAA gene. In another embodiment, the individual suffering from methylmalonic aciduria has a mutation, or has at least one mutation, or has two or more mutations, in the MMAB gene. In another embodiment, the individual with isovaleric aciduria has a mutation, one or more mutations, or two or more mutations in the IVD gene.

In one embodiment, the individual suffering from methylmalonic aciduria, isovaleric aciduria, or another organic aciduria, such as an individual suffering from methylmalonic aciduria, has one or more symptoms selected from the group consisting of: progressive encephalopathy, dehydration, feeding problems, developmental delays, failure to thrive, lethargy, recurrent yeast infections, seizures, emesis, kidney disease, kidney failure, pancreatitis, coma, brain abnormalities, ventricular dilation, cortical atrophy, periventricular white matter abnormality, thinning of the corpus callosum, subcortical white matter abnormality, cerebellar atrophy, basal ganglionic calcification, and myelination delay.

In one embodiment, administration of a therapeutically effective amount of one or more of alpha-tocotrienol quinone, alpha-tocotrienol hydroquinone, beta-tocotrienol quinone, beta-tocotrienol hydroquinone, gamma-tocotrienol quinone, gamma-tocotrienol hydroquinone, delta-tocotrienol quinone, or delta-tocotrienol hydroquinone, such as a therapeutically effective amount of alpha-tocotrienol quinone, to an individual suffering from methylmalonic aciduria, isovaleric aciduria, or another organic aciduria, such as an individual suffering from methylmalonic aciduria, alleviates one or more symptoms selected from the group consisting of: progressive encephalopathy, dehydration, feeding problems, developmental delays, failure to thrive, lethargy, recurrent yeast infections, seizures, emesis, kidney disease, kidney failure, pancreatitis, coma, brain abnormalities, ventricular dilation, cortical atrophy, periventricular white matter abnormality, thinning of the corpus callosum, subcortical white matter abnormality, cerebellar atrophy, basal ganglionic calcification, and myelination delay.

In one embodiment, the compound used in treatment is able to cross the blood-brain barrier to provide a therapeutic level of compound in the central nervous system, as measured by the concentration of compound in the cerebrospinal fluid. In one embodiment, the compound used in treatment crosses the blood-brain barrier by transmembrane diffusion. In another embodiment, the compound used in treatment is administered into the cerebrospinal fluid.

In one embodiment, the compound for use is administered to the patient in an amount such that the concentration of the compound in the cerebrospinal fluid of the patient is between about 0.1 ng/ml and about 10 ng/ml. In one embodiment, the compound for use is administered to the patient in an amount such that the concentration of the compound in the cerebrospinal fluid of the patient is between about 0.2 ng/ml and about 5 ng/ml. In one embodiment, the compound for use is administered to the patient in an amount such that the concentration of the compound in the cerebrospinal fluid of the patient is between about 0.4 ng/ml and about 3 ng/ml. In one embodiment, the compound for use is administered to the patient in an amount such that the concentration of the compound in the cerebrospinal fluid of the patient is between about 0.5 ng/ml and about 2 ng/ml. In one embodiment, the compound for use is administered to the patient in an amount such that the concentration of the compound in the cerebrospinal fluid of the patient is between about 0.75 ng/ml and about 2 ng/ml. In one embodiment, the compound for use is administered to the patient in an amount such that the concentration of the compound in the cerebrospinal fluid of the patient is between about 1 ng/ml and about 2 ng/ml. In one embodiment, the compound for use is administered to the patient in an amount such that the concentration of the compound in the cerebrospinal fluid of the patient is between about 0.75 ng/ml and about 1.5 ng/ml. In one embodiment, the compound used for for use is administered to the patient in an amount such that the concentration of the compound in the cerebrospinal fluid of the patient is between about 1 ng/ml and about 1.5 ng/ml. In one embodiment, the compound for use s administered to the patient in an amount such that the concentration of the compound in the cerebrospinal fluid of the patient is about 1.3 ng/ml.

In one embodiment, the compound for use is administered to the patient in an amount such that the concentration of the compound in the cerebrospinal fluid of the patient is at or above about 0.1 ng/ml. In one embodiment, the compound for use is administered to the patient in an amount such that the concentration of the compound in the cerebrospinal fluid of the patient is at or above about 0.2 ng/ml. In one embodiment, the compound for use is administered to the patient in an amount such that the concentration of the compound in the cerebrospinal fluid of the patient is at or above about 0.3 ng/ml. In one embodiment, the compound for use is administered to the patient in an amount such that the concentration of the compound in the cerebrospinal fluid of the patient is at or above about 0.4 ng/ml. In one embodiment, the compound for use is administered to the patient in an amount such that the concentration of the compound in the cerebrospinal fluid of the patient is at or above about 0.5 ng/ml. In one embodiment, the compound for use is administered to the patient in an amount such that the concentration of the compound in the cerebrospinal fluid of the patient is at or above about 0.75 ng/ml. In one embodiment, the compound for use is administered to the patient in an amount such that the concentration of the compound in the cerebrospinal fluid of the patient is at or above about 1 ng/ml.

In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is between about 0.1 ng/ml and about 10 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is between about 0.2 ng/ml and about 5 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is between about 0.4 ng/ml and about 3 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is between about 0.5 ng/ml and about 2 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is between about 0.75 ng/ml and about 2 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is between about 1 ng/ml and about 2 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is between about 0.75 ng/ml and about 1.5 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is between about 1 ng/ml and about 1.5 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is about 1.3 ng/ml.

In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is at or above about 0.1 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is at or above about 0.2 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is at or above about 0.3 ng/ml. In one embodiment, the for use is alpha-tocotrienol quinone, and the alpha-tocotrienol quinone is administered to the patient in an amount such that the concentration of alpha-tocotrienol quinone in the cerebrospinal fluid of the patient is at or above about 0.4 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is at or above about 0.5 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is at or above about 0.75 ng/ml. In one embodiment, the compound for use is alpha-tocotrienol quinone, and the alpha-tocotrienol quinone is administered to the patient in an amount such that the concentration of alpha-tocotrienol quinone in the cerebrospinal fluid of the patient is at or above about 1 ng/ml.

In one embodiment, the compound for use is administered to the patient in an amount such that the concentration of the compound in the plasma of the patient is between about 1 ng/ml and about 5,000 ng/ml. In one embodiment, the compound for use is administered to the patient in an amount such that the concentration of the compound in the plasma of the patient is between about 10 ng/ml and about 2,000 ng/ml. In one embodiment, the compound for use is administered to the patient in an amount such that the concentration of the compound in the plasma of the patient is between about 10 ng/ml and about 2,000 ng/ml. In one embodiment, the compound for use is administered to the patient in an amount such that the concentration of the compound in the plasma of the patient is between about 10 ng/ml and about 1,000 ng/ml. In one embodiment, the compound for use is administered to the patient in an amount such that the concentration of the compound in the plasma of the patient is between about 10 ng/ml and about 500 ng/ml. In one embodiment, the compound for use is administered to the patient in an amount such that the concentration of the compound in the plasma of the patient is between about 10 ng/ml and about 250 ng/ml. In one embodiment, the compound for use is administered to the patient in an amount such that the concentration of the compound in the plasma of the patient is between about 10 ng/ml and about 150 ng/ml. In one embodiment, the compound for use is administered to the patient in an amount such that the concentration of the compound in the plasma of the patient is between about 10 ng/ml and about 100 ng/ml. In one embodiment, the compound for use is administered to the patient in an amount such that the concentration of the compound in the plasma of the patient is about 50 ng/ml.

In one embodiment, the compound for use is administered to the patient in an amount such that the concentration of the compound in the plasma of the patient is at or above about 1 ng/ml. In one embodiment, the compound for use is administered to the patient in an amount such that the concentration of the compound in the plasma of the patient is at or above about 5 ng/ml. In one embodiment, the compound for use is administered to the patient in an amount such that the concentration of the compound in the plasma of the patient is at or above about 10 ng/ml. In one embodiment, the compound for use is administered to the patient in an amount such that the concentration of the compound in the plasma of the patient is at or above about 25 ng/ml. In one embodiment, the compound for use is administered to the patient in an amount such that the concentration of the compound in the plasma of the patient is at or above about 50 ng/ml. In one embodiment, the compound for use is administered to the patient in an amount such that the concentration of the compound in the plasma of the patient is at or above about 75 ng/ml. In one embodiment, the compound for use is administered to the patient in an amount such that the concentration of the compound in the plasma of the patient is at or above about 100 ng/ml.

In one embodiment, the compound for use is alpha-tocotrienol quinone, and the alpha-tocotrienol quinone is administered to the patient in an amount such that the concentration of alpha-tocotrienol quinone in the plasma of the patient is between about 1 ng/ml and about 5,000 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is between about 10 ng/ml and about 2,000 ng/ml. In one embodiment, the compound used for treatment for use is alpha-tocotrienol quinone, and the alpha-tocotrienol quinone is administered to the patient in an amount such that the concentration of alpha-tocotrienol quinone in the plasma of the patient is between about 10 ng/ml and about 2,000 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is between about 10 ng/ml and about 1,000 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is between about 10 ng/ml and about 500 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is between about 10 ng/ml and about 250 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is between about 10 ng/ml and about 150 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is between about 10 ng/ml and about 100 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is about 50 ng/ml.

In one embodiment, the compound for use is alpha-tocotrienol quinone, and the alpha-tocotrienol quinone is administered to the patient in an amount such that the concentration of alpha-tocotrienol quinone in the plasma of the patient is at or above about 1 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is at or above about 5 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is at or above about 10 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is at or above about 25 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is at or above about 50 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is at or above about 75 ng/ml. In one embodiment, the compound for use is alpha-tocotrienol quinone, and the alpha-tocotrienol quinone is administered to the patient in an amount such that the concentration of alpha-tocotrienol quinone in the plasma of the patient is at or above about 100 ng/ml.

In one embodiment, the compound for use in a method of treating methylmalonic aciduria, isovaleric aciduria, or another organic aciduria is selected from the group consisting of alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, or any combination of two or more of the foregoing compounds, and is formulated in a pharmaceutical preparation suitable for administration via feeding tube, feeding syringe, or gastrostomy. In another embodiment, the compound for use in a method of treating methylmalonic aciduria, isovaleric aciduria, or another organic aciduria is selected from the group consisting of alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, or any combination of two or more of the foregoing compounds, and is formulated in a pharmaceutical preparation comprising one or more vegetable-derived oils, such as sesame oil, and/or one or more animal-derived oils, and/or one or more fish-derived oils. In another embodiment, the compound for use in a method of treating methylmalonic aciduria, isovaleric aciduria, or another organic aciduria is alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, or any combination of two or more of the foregoing compounds, and is formulated in a pharmaceutical preparation comprising one or more vegetable-derived oils, such as sesame oil, and/or one or more animal-derived oils, and/or one or more fish-derived oils, where the pharmaceutical preparation is suitable for administration via feeding tube, feeding syringe, or gastrostomy.

For all of the compounds for use in methods described herein which use a tocotrienol quinone, the quinone form can also be used in its reduced (hydroquinone, 1,4-benzenediol) form when desired. Likewise, the hydroquinone form can also be used in its oxidized (quinone) form when desired.

For all of the compounds and methods described herein, the invention also encompasses the use in treatment of the compounds and methods disclosed. The invention also encompasses the use of the compounds described herein for preparation of a medicament for use in treating methylmalonic aciduria. The invention also encompasses the use of the compounds described herein for preparation of a medicament for use in treating isovaleric aciduria or another organic aciduria.

The present invention comprises multiple aspects, features and embodiments, where such multiple aspects, features and embodiments can be combined and permuted in any desired manner. These and other aspects, features and embodiments of the present invention will become evident upon reference to the remainder of this application, including the following detailed description.

### BRIEF DESCRIPTION OF THE DRAWINGS

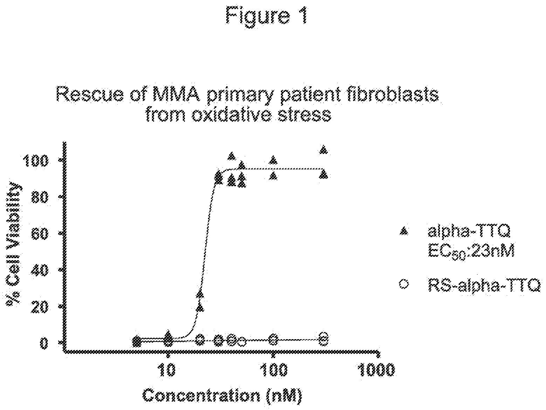

Figure 1 depicts the results of a cell viability assay for MMA cells in the presence of alpha-tocotrienol quinone (αTTQ) and redox-silent alpha-tocotrienol quinone (αTTQ-RS).

### DETAILED DESCRIPTION OF THE INVENTION

The present invention relates to a compound selected from the group consisting of tocotrienol quinones and tocotrienol hydroquinones for use in a method of treating methylmalonic aciduria, isovaleric aciduria, or another organic aciduria in an individual.

In one aspect, tocotrienol quinones are contemplated for use in treatment, including alpha-tocotrienol quinone, beta-tocotrienol quinone, gamma-tocotrienol quinone, and delta-tocotrienol quinone. In another aspect, alpha-tocotrienol quinone is contemplated for use in treatment. Structures of tocotrienol quinones are given in Table 1 below. The tocotrienol quinones with the naturally occurring tocotrienol configuration are used in one embodiment of the invention, but other stereoisomers and/or mixtures of stereoisomers in any ratio, such as racemic mixtures, can also be used in the invention.

Tocotrienol quinones can be used in their oxidized form, as shown in Table 1, or can be used in their reduced hydroquinone form, as shown in Table 2. The quinone (cyclohexadienedione) form and hydroquinone (benzenediol) form are readily interconverted with appropriate reagents. The quinone can be treated in a biphasic mixture of an ethereal solvent with a basic aqueous solution of Na₂S₂O₄ (Vogel, A.I. et al. Vogel's Textbook of Practical Organic Chemistry, 5th Edition, Prentice Hall: New York, 1996; Section 9.6.14 Quinones, "Reduction to the Hydroquinone"). Standard workup in the absence of oxygen yields the desired hydroquinone. The hydroquinone form can be oxidized to the quinone form with oxidizing agents such as ceric ammonium nitrate (CAN) or ferric chloride. The quinone and hydroquinone forms are also readily interconverted electrochemically, as is well known in the art. See, e.g., Section 33.4 of Streitweiser & Heathcock, Introduction to Organic Chemistry, New York: Macmillan, 1976.

**Table 1**

| Tocotrienol quinones | | | | |
|---|---|---|---|---|
| 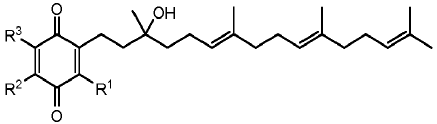 | | R¹ | R² | R³ |
| Alpha-tocotrienol quinone | 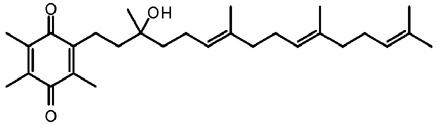 | methyl | methyl | methyl |
| Beta-tocotrienol quinone | 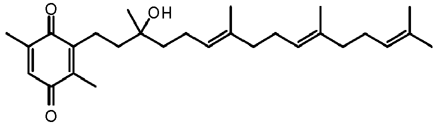 | methyl | H | methyl |
| Gamma-tocotrienol quinone | 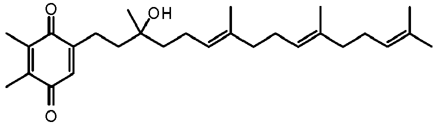 | H | methyl | methyl |
| Delta-tocotrienol quinone | 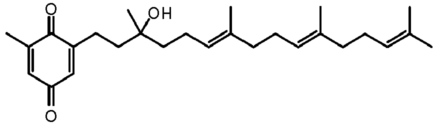 | H | H | methyl |

**Table 2**

| Tocotrienol hydroquinones | | | | |
|---|---|---|---|---|
| 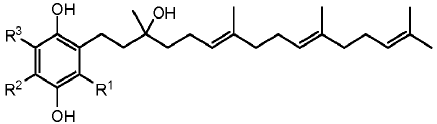 | | R¹ | R² | R³ |
| Alpha-tocotrienol hydro quinone | 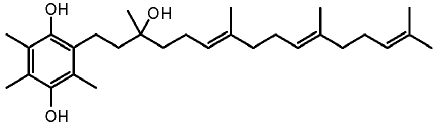 | methyl | methyl | methyl |
| Beta-tocotrienol hydro quinone | 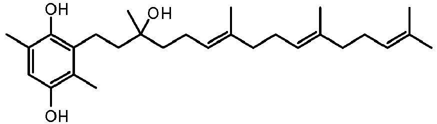 | methyl | H | methyl |
| Gamma-tocotrienol hydroquinone | 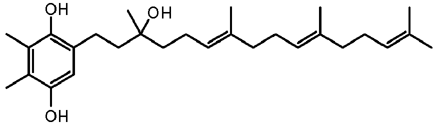 | H | methyl | methyl |
| Delta-tocotrienol hydro quinone | 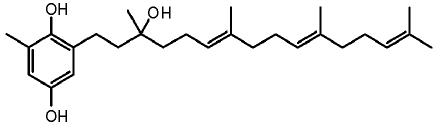 | H | H | methyl |

By "individual," "subject," or "patient," is meant a mammal, preferably a human.

"Treating" a disease with the compounds and methods discussed herein is defined as administering one or more of the compounds discussed herein, with or without additional therapeutic agents, in order to reduce or eliminate either the disease or one or more symptoms of the disease, or to retard the progression of the disease or of one or more symptoms of the disease, or to reduce the severity of the disease or of one or more symptoms of the disease. "Suppression" of a disease with the compounds and methods discussed herein is defined as administering one or more of the compounds discussed herein, with or without additional therapeutic agents, in order to suppress the clinical manifestation of the disease, or to suppress the manifestation of adverse symptoms of the disease. The distinction between treatment and suppression is that treatment occurs after adverse symptoms of the disease are manifest in a subject, while suppression occurs before adverse symptoms of the disease are manifest in a subject. Suppression may be partial, substantially total, or total.

Because methylmalonic aciduria and isovaleric aciduria or another organic aciduria are due to genetic mutations, genetic screening can be used to identify patients at risk of the disease. Methylmalonic aciduria can arise from mutations in one or more of the MUT, MMAA, or MMAB genes. The compounds disclosed herein can be administered to, and the methods disclosed herein can be used to treat, asymptomatic patients with mutations in one or more of the MUT, MMAA, or MMAB genes, who are at risk of developing the clinical symptoms of the disease, in order to suppress the appearance of any adverse symptoms or lessen the severity of symptoms that may occur. The compounds disclosed herein can be administered to, and the methods disclosed herein can be used to treat, symptomatic patients with mutations in one or more of the MUT, MMAA, or MMAB genes, in order to treat the disease. Isovaleric aciduria can arise from mutations in the IVD gene. The compounds disclosed herein can be administered to, and the methods disclosed herein can be used to treat, asymptomatic patients with mutations in the IVD gene, who are at risk of developing the clinical symptoms of the disease, in order to suppress the appearance of any adverse symptoms or lessen the severity of symptoms that may occur. The compounds disclosed herein can be administered to, and the methods disclosed herein can be used to treat, symptomatic patients with mutations in the IVD gene, in order to treat the disease.

"Therapeutic use" of the compounds discussed herein is defined as using one or more of the compounds discussed herein to treat or suppress a disease, as defined above. A "therapeutically effective amount" of a compound is an amount of the compound, which, when administered to a subject, is sufficient to reduce or eliminate either a disease or one or more symptoms of a disease, or to retard the progression of a disease or of one or more symptoms of a disease, or to reduce the severity of a disease or of one or more symptoms of a disease, or to suppress the clinical manifestation of a disease, or to suppress the manifestation of adverse symptoms of a disease. A therapeutically effective amount can be given in one or more administrations.

While the compounds described herein can occur and can be used as the neutral (non-salt) compound, the description is intended to embrace all salts of the compounds described herein, as well as methods of using such salts of the compounds. In one embodiment, the salts of the compounds comprise pharmaceutically acceptable salts. Pharmaceutically acceptable salts are those salts which can be administered as drugs or pharmaceuticals to humans and/or animals and which, upon administration, retain at least some of the biological activity of the free compound (neutral compound or non-salt compound). The desired salt of a basic compound may be prepared by methods known to those of skill in the art by treating the compound with an acid. Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. Salts of basic compounds with amino acids, such as aspartate salts and glutamate salts, can also be prepared. The desired salt of an acidic compound can be prepared by methods known to those of skill in the art by treating the compound with a base. Examples of inorganic salts of acid compounds include, but are not limited to, alkali metal and alkaline earth salts, such as sodium salts, potassium salts, magnesium salts, and calcium salts; ammonium salts; and aluminum salts. Examples of organic salts of acid compounds include, but are not limited to, procaine, dibenzylamine, N-ethylpiperidine, N,N-dibenzylethylenediamine, and triethylamine salts. Salts of acidic compounds with amino acids, such as lysine salts, can also be prepared.

The description of compounds herein also includes all stereoisomers of the compounds, including diastereomers and enantiomers, and mixtures of stereoisomers in any ratio, including, but not limited to, racemic mixtures. Unless stereochemistry is explicitly indicated in a structure, the structure is intended to embrace all possible stereoisomers of the compound depicted. If stereochemistry is explicitly indicated for one portion or portions of a molecule, but not for another portion or portions of a molecule, the structure is intended to embrace all possible stereoisomers for the portion or portions where stereochemistry is not explicitly indicated.

The compounds can be administered in prodrug form. Prodrugs are derivatives of the compounds, which are themselves relatively inactive but which convert into the active compound when introduced into the subject in which they are used by a chemical or biological process in vivo, such as an enzymatic conversion. Suitable prodrug formulations include, but are not limited to, peptide conjugates of the compounds disclosed herein and esters of compounds disclosed herein. Further discussion of suitable prodrugs is provided in H. Bundgaard, Design of Prodrugs, New York: Elsevier, 1985; in R. Silverman, The Organic Chemistry of Drug Design and Drug Action, Boston: Elsevier, 2004; in R.L. Juliano (ed.), Biological Approaches to the Controlled Delivery of Drugs (Annals of the New York Academy of Sciences, v. 507), New York: New York Academy of Sciences, 1987; and in E.B. Roche (ed.), Design of Biopharmaceutical Properties Through Prodrugs and Analogs (Symposium sponsored by Medicinal Chemistry Section, APhA Academy of Pharmaceutical Sciences, November 1976 national meeting, Orlando, Florida), Washington : The Academy, 1977.

### Monitoring Treatment Efficacy Using Biomarkers

Several metabolic biomarkers can be used to monitor the efficacy of compounds in treatment of methylmalonic aciduria and isovaleric aciduria or another organic aciduria. These biomarkers include, but are not limited to, lactic acid (lactate) levels, either in whole blood, plasma, cerebrospinal fluid, or cerebral ventricular fluid; pyruvic acid (pyruvate) levels, either in whole blood, plasma, cerebrospinal fluid, or cerebral ventricular fluid; lactate/pyruvate ratios, either in whole blood, plasma, cerebrospinal fluid, or cerebral ventricular fluid; phosphocreatine levels, NADH (NADH +H⁺) or NADPH (NADPH+H⁺) levels; NAD or NADP levels; ATP levels; anaerobic threshold; reduced coenzyme Q (CoQ^{red}) levels; oxidized coenzyme Q (CoQ^{ox}) levels; total coenzyme Q (CoQ^{tot}) levels; oxidized cytochrome c levels; reduced cytochrome c levels; oxidized cytochrome c/reduced cytochrome c ratio; acetoacetate levels, β-hydroxy butyrate levels, acetoacetate/β-hydroxy butyrate ratio, 8-hydroxy-2'-deoxyguanosine (8-OHdG) levels; levels of reactive oxygen species; and levels of oxygen consumption (VO2), levels of carbon dioxide output (VCO2), and respiratory quotient (VCO2/VO2). Exercise intolerance can also be used as an indicator of the efficacy of compounds in treatment of methylmalonic aciduria and isovaleric aciduria or another organic aciduria. Several of these clinical markers are measured routinely in exercise physiology laboratories, and provide convenient assessments of the metabolic state of a subject.

Monitoring evaluation of redox status in various organs, particularly the brain by imaging can be assessed with tracer techniques such as HMPAO, Tc99m-HMPAO, or other used imaging agents. Serum levels of glutathione are assessed *in vivo* by HMPAO SPECT imaging using Tc99m-HMPAO. See, e.g., Atkuri et al., Proc. Natl. Acad. Sci. (USA) 106:3941-3945 (2009), where individuals with mitochondrial dysfunction were shown to have glutathione deficiencies, as well as lowered plasma citrulline levels (citrulline can also be used as a marker for treatment efficacy).

Pyruvate, a product of the metabolism of glucose, is removed by reduction to lactic acid in an anaerobic setting; the degree to which this process occurs is dependent on the function of the mitochondrial respiratory chain. Dysfunction of the respiratory chain can lead to an abnormally high conversion of pyruvate to lactate, supported by the elevated lactate/pyruvate ratios observed in mitochondrial cytopathies (Scriver CR. The metabolic and molecular bases of inherited disease. 7th ed. New York: McGraw-Hill, Health Professions Division; 1995; Munnich A, Rustin P, Rotig A, et al. Clinical aspects of mitochondrial disorders. J Inherit Metab Dis. 1992; 15(4):448-455). Blood lactate/pyruvate ratio is, therefore, widely used as a noninvasive test for detection of mitochondrial cytopathies and toxic mitochondrial myopathies. (See Chariot P, Ratiney R, Ammi-Said M, Herigault R, Adnot S, Gherardi R. Optimal handling of blood samples for routine measurement of lactate and pyruvate. Arch Pathol Lab Med. Jul 1994;118(7):695-697; Chariot P, Monnet I, Mouchet M, et al. Determination of the blood lactate:pyruvate ratio as a noninvasive test for the diagnosis of zidovudine myopathy. Arthritis Rheum. Apr 1994;37(4):583-586.) Total concentration levels of lactate and total concentration levels of pyruvate that are elevated above the normal range are also observed in methylmalonic aciduria, and those elevated total concentrations can serve as additional biomarkers in addition to the elevated lactate/pyruvate ratio. Another biomarker which can be monitored is CoQ₁₀ concentration.

Biomarkers and techniques for measurement of biomarkers that can be used to monitor the efficacy of treatment include, but are not limited to:
*Magnetic resonance spectroscopy:* Brain lactate measurement and quantification directly reflect cellular electron balance and indirectly reflect energy production. Magnetic resonance spectroscopy can be used to assess metabolic parameters of the brain with a focus on lactate, i.e., central nervous system (CNS) concentration of lactate and lactate/pyruvate ratio. MRS has been used to measure lactate using proton MRS (1H-MRS) (Kaufmann et al., Neurology 62(8):1297-302 (2004)). Phosphorous MRS (31P-MRS) has been used to demonstrate low levels of cortical phosphocreatine (PCr) (Matthews et al., Ann. Neurol. 29(4):435-8 (1991)), and a delay in PCr recovery kinetics following exercise in skeletal muscle (Matthews et al., Ann. Neurol. 29(4):435-8 (1991); Barbiroli et al., J. Neurol. 242(7):472-7 (1995); Fabrizi et al., J. Neurol. Sci. 137(1):20-7 (1996)).
   Proton Magnetic Resonance Spectroscopy (MRS) is used to measure the levels of different metabolic compounds in the brain of mitochondrial patients, that emit a unique resonance frequency expressed as chemical shifts in parts per million (ppm). Patients with mitochondrial disease are evaluated for lactate, N-acetyl aspartate (NAA), succinate, total creatine, choline (Cho) and myo-inositol. Lactate is not detected in normal patients; however, if metabolism shifts to anaerobic glycolysis in mitochondrial respiratory chain deficiencies, lactate levels increase (see Barkovich and et al, AJNR Am. J. Neuroradiol.(1993) 14, (5) 1119-1137). One of the best biomarkers for neuronal integrity is NAA which is localized to neurons and dendrites (see Clark, JB, Dev. Neurosci. (1998), 20 (4-5)271-276). Reductions of NAA levels when normalized to creatine are seen in mitochondrial disease patients. The signal for choline (Cho) includes free choline, phosphoryl choline and phosphatidylcholine which constitute myelin. Cho elevations reflect membrane turnover and demyelination. Deficient respiratory chain activity produces increases in succinate concentration detectable by this method (see Brockmann et al., Ann. Neurol. (2002)52 (1) 38-45). Proton MRS obtained over conventional MRI, provides additional information through visualization of metabolic changes.
*Lactic acid (lactate) levels:* Brain lactate measurement and quantification directly reflect cellular electron balance and indirectly reflect energy production. Lactate levels can be measured by taking samples of appropriate bodily fluids such as whole blood, plasma, or cerebrospinal fluid. Using magnetic resonance, lactate levels can be measured in virtually any volume of the body desired, such as the brain. Whole blood, plasma, and cerebrospinal fluid lactate levels can be measured by commercially available equipment such as the YSI 2300 STAT Plus Glucose & Lactate Analyzer (YSI Life Sciences, Ohio).
*NAD, NADP, NADH and NADPH levels:* Measurement of NAD, NADP, NADH (NADH +H⁺) or NADPH (NADPH+H⁺) can be measured by a variety of fluorescent, enzymatic, or electrochemical techniques, e.g., the electrochemical assay described in US 2005/0067303.
*Oxygen consumption (vO₂ or VO2), carbon dioxide output (vCO₂ or VCO2), and respiratory quotient (RQ* = *VCO2*/*VO2):* vO₂ is usually measured either while resting (resting vO₂) or at maximal exercise intensity (vO₂ max). Optimally, both values will be measured. However, for severely disabled patients, measurement of vO₂ max may be impractical. Measurement of both forms of vO₂ is readily accomplished using standard equipment from a variety of vendors, e.g., Korr Medical Technologies, Inc. (Salt Lake City, Utah). VCO2 can also be readily measured, and the ratio of VCO2 to VO2 under the same conditions (VCO2/VO2, either resting or at maximal exercise intensity) provides the respiratory quotient (RQ).
*Other oxygen metabolism deficiencies:* Other problems with oxygen metabolism which can be measured include deficit in peripheral oxygen extraction (A-VO2 difference) and an enhanced oxygen delivery (hyperkinetic circulation) (Taivassalo et al., Brain 126(Pt 2):413-23 (2003)). This can be demonstrated by a lack of exercise induced deoxygenation of venous blood with direct AV balance measurements (Taivassalo et al., Ann. Neurol. 51(1):38-44 (2002)) and non-invasively by near infrared spectroscopy (Lynch et al., Muscle Nerve 25(5):664-73 (2002); van Beekvelt et al., Ann. Neurol. 46(4):667-70 (1999)).
*Oxidized Cytochrome c, reduced Cytochrome c, and ratio of oxidized Cytochrome c to reduced Cytochrome c:* Cytochrome c parameters, such as oxidized cytochrome c levels (Cyt Cₒₓ), reduced cytochrome c levels (Cyt C_{red}), and the ratio of oxidized cytochrome c/reduced cytochrome c ratio (Cyt Cₒₓ)/(Cyt C_{red}), can be measured by *in vivo* near infrared spectroscopy. See, e.g., Rolfe, P., "In vivo near-infrared spectroscopy," Ann. Rev. Biomed. Eng. 2:715-54 (2000) and Strangman et al., "Non-invasive neuroimaging using near-infrared light" Biol. Psychiatry 52:679-93 (2002).
*Exercise tolerance*/*Exercise intolerance:* Exercise intolerance is defined as "the reduced ability to perform activities that involve dynamic movement of large skeletal muscles because of symptoms of dyspnea or fatigue" (Piña et al., Circulation 107:1210 (2003)). Exercise intolerance is often accompanied by myoglobinuria, due to breakdown of muscle tissue and subsequent excretion of muscle myoglobin in the urine. Various measures of exercise intolerance can be used, such as time spent walking or running on a treadmill before exhaustion, time spent on an exercise bicycle (stationary bicycle) before exhaustion, and similar tests.
*Acetoacetate*/*3-hydroxybutyrate (acetoacetate*/*β-hydroxybutyrate) ratio:* Changes in the redox state of liver mitochondria can be investigated by measuring the arterial ketone body ratio (acetoacetate/3-hydroxybutyrate: AKBR) (Ueda et al., J. Cardiol. 29(2):95-102 (1997)).
*8-hydroxy-2'-deoxyguanosine (8-OHdG):* Urinary excretion of 8-hydroxy-2'-deoxyguanosine (8-OHdG) often has been used as a biomarker to assess the extent of repair of ROS-induced DNA damage in both clinical and occupational settings (Erhola et al., FEBS Lett. 409(2):287-91 (1997); Honda et al., Leuk. Res. 24(6):461-8 (2000); Pilger et al., Free Radic. Res. 35(3):273-80 (2001); Kim et al. Environ Health Perspect 112(6):666-71 (2004)).
*Routine plasma analytes:* Blood ketone body ratios, including lactate: pyruvate and beta-hydroxy butyrate:acetoacetate, reflect electron balance. Alterations in these ratios can be used to assess systemic metabolic function. Increased blood lactate, increased blood pyruvate, increased blood alanine, and blood pH (to check for metabolic acidosis) can also be monitored.
*Other blood, metabolic, or enzymatic biomarkers:* patients can be monitored for an increase in the number of white blood cells, and for cytochrome c oxidase deficiency.
*Routine measures of cardiac function:* Mitochondrial diseases are frequently characterized by altered heart function. 12-lead ECG can be employed to measure QT/QTc. Transthoracic echocardiography can be used to assess dynamic cardiac function.
*Measurements of brainstem function:* brainstem auditory evoked response (BAER), somatosensory-evoked potentials (SEP or SSEP), blink reflex, and polysomnography (PSG) can be monitored in patients to assess brainstem function.
*Other reflexes:* Babinski test (Babinski reflex, Babinski sign), which can indicate motor neuron damage.
*Metabolomic analysis of plasma and urine:* Plasma and urine analysis can be performed on the patient, and can include measurement of the following organic acids: methyl malonic acid, isovaleric acid, lactic acid, pyruvic acid, succinic acid, fumaric acid, 2-ketoglutaric acid, 3-OH butyric acid, acetoacetic acid, 2-keto-3-methylvaleric acid, 2-keto-isocaproic acid, 2-keto-isovaleric acid, ethylmalonic acid, adipic acid, suberic acid, sebacic acid, 4-OH-phenylacetic acid, 4-OH-phenyllactic acid, 4-OH-phenylpyruvic acid, succinylacetone, and creatinine. Urine analysis performed on the patient can also include measurement of the following amino acids: proline, glutamine, threonine, serine, glutamic acid, arginine, glycine, alanine, histidine, lysine, valine, asparagine, methionine, phenylalanine, isoleucine, leucine, tyrosine, hydroxyproline, creatinine, aspartic acid, cysteine, ornithine, citrulline, homocysteine, and taurine. In a panel of metabolic analytes, the following can be measured: sodium, potassium, chloride, bicarbonate, anion gap, glucose (serum), urea nitrogen (blood), creatinine, calcium, bilirubin, aspartate amino transferase, alanine amino transferase, alkaline phosphatase, total protein (serum), albumin (serum), and hemolysis index. Recently, the Critical Path Initiative has put forth a battery of biomarkers to predict drug toxicity that can also reflect renal mitochondrial function. Finally, Haas, et al. Mol Genet Metab. (2008) 94(1):16-37 describes various tests, such as MRS-based biochemical analysis.

### Methylmalonic aciduria, isovaleric aciduria, and other organic acidurias: Symptoms Amenable to Treatment

Methylmalonic aciduria progressive encephalopathy, dehydration, feeding problems, developmental delays, failure to thrive, lethargy, recurrent yeast infections, seizures, emesis, kidney disease, kidney failure, pancreatitis, coma, brain abnormalities, ventricular dilation, cortical atrophy, periventricular white matter abnormality, thinning of the corpus callosum, subcortical white matter abnormality, cerebellar atrophy, basal ganglionic calcification, and myelination delay

Symptoms of isovaleric aciduria or another organic aciduria are similar to those of methylmalonic aciduria.

In one embodiment, the compounds for use of the invention can alleviate one or more symptoms of methylmalonic aciduria, isovaleric aciduria, or another organic aciduria, including progressive encephalopathy, dehydration, feeding problems, developmental delays, failure to thrive, lethargy, recurrent yeast infections, seizures, emesis, kidney disease, kidney failure, pancreatitis, coma, brain abnormalities, ventricular dilation, cortical atrophy, periventricular white matter abnormality, thinning of the corpus callosum, subcortical white matter abnormality, cerebellar atrophy, basal ganglionic calcification, and myelination delay.

Standard motor function tests can be used to assess many of these symptoms, including tests used by physical therapists, occupational therapists, and rehabilitation medicine specialists to assess patient function. As many patients presenting with methylmalonic aciduria, isovaleric aciduria, or another organic aciduria are young, age-appropriate tests are used.

There are several known assessment products for pediatricians to evaluate children. For physical abilities, the Pediatric Evaluation of Disability Inventory (PEDI) can be used (see Haley, S. M., Coster, W. J., Ludlow, L. H., Haltiwanger, J. T., & Andrellos, P. J. (1992). Pediatric Evaluation of Disability Inventory: Development, Standardization, and Administration Manual, Version 1.0. Boston , MA : Trustees of Boston University , Health and Disability Research Institute); PEDI enables evaluation of functional disabilities using standardized score forms. The PEDI can be used to assess key functional capabilities and performance in children ages six months to seven years, and to evaluate older children whose functional abilities are lower than those of seven-year-olds without disabilities. PEDI can be used to identify functional deficits and monitor treatment progress.

For neuro-psychiatric evaluation, the NEPSY-II assessment (Korkman, Marit; Kirk, Ursula; & Kemp, Sally. (2007) NEPSY-II-Second Edition, San Antonio, Texas: Pearson) can be used to gauge neuropsychological development. Testing in children 3-4 years of age can assess six functional domains: attention and executive functions; language and communication; sensorimotor functions; visuospatial functions; learning and memory; and social perception.

Additionally Wolf N.I. et al., "Mitochondrial disorders: a proposal for consensus diagnostic criteria in infants and children," Neurology (2002) 59 (9) 1402-1405 also describes diagnostic criteria in infants and children with mitochondrial diseases.

A scale to monitor progression and treatment of mitochondrial diseases in children, commonly known as the Newcastle Paediatric Mitochondrial Disease Scale (NPMDS), monitors the biophysical markers of disease progression. The scale is based around four domains: current function; system-specific involvement; current clinical assessment; and quality of life, as described by C. Phoenix et al, "A scale to monitor progression and treatment of mitochondrial disease in children," Neuromuscular Disorders (2006) 16 814-820.

### Mutations causing methylmalonic aciduria

Several mutations in genes involved in metabolism are implicated in methylmalonic aciduria. These genes include MUT, MMAA, and MMAB. A gene implicated in isovaleric academia is IVD.

### Dosages

The compounds for use of the invention can be administered in various amounts. Examples of daily dosages which can be used are an effective amount within the dosage range of about 0.1 mg/kg to about 300 mg/kg body weight, or within about 0.1 mg/kg to about 100 mg/kg body weight, or within about 0.1 mg/kg to about 80 mg/kg body weight, or within about 0.1 mg/kg to about 50 mg/kg body weight, or within about 0.1 mg/kg to about 30 mg/kg body weight, or within about 0.1 mg/kg to about 10 mg/kg body weight, or within about 1.0 mg/kg to about 80 mg/kg body weight, or within about 1.0 mg/kg to about 50 mg/kg body weight, or within about 1.0 mg/kg to about 30 mg/kg body weight, or within about 1.0 mg/kg to about 10 mg/kg body weight, or within about 10 mg/kg to about 80 mg/kg body weight, or within about 50 mg/kg to about 150 mg/kg body weight, or within about 100 mg/kg to about 200 mg/kg body weight, or within about 150 mg/kg to about 250 mg/kg body weight, or within about 200 mg/kg to about 300 mg/kg body weight, or within about 250 mg/kg to about 300 mg/kg body weight, or about 0.1, about 5, about 10, about 15, about 20, about 25, about 30, about 40, about 50, about 60, about 70, about 75, about 80, about 90, about 100, about 125, about 150, about 175, about 200, about 225, about 250, about 275, about 300, about 325, about 350, about 375, about 400, about 425, about 450, about 500, about 550, about 600, about 650, about 700, about 750, about 800, about 850, about 900, about 950, or about 1000 mg total. The compound(s) may be administered in a single daily dose, or the total daily dosage may be administered in divided dosages of two, three or four times daily. These dosages can be administered long term, for example, over months, years, or even over the entire lifetime of the patient.

The particular dosage appropriate for a specific patient is determined by dose titration. For example, animal studies of alpha-tocotrienol quinone administration have shown that in rats, at 10 mg/kg, bioavailability is high (∼90%), Cₘₐₓ = 931 ng/mL, Tₘₐₓ = 3.5 h and t_{1/2} = 3.5 h. There is less than dose-proportionality since for an increase in doses of 2.4 : 6 : 10 : 20 there is only an increase in AUCs of 1.5 : 2.8 : 4.0 : 6.7. This lack of dose-proportionality may be due to decreased absorption since there is no change in t_{1/2} over dose range. Alpha-tocotrienol quinone tested in rats was safe when given acutely up to 2000 mg/kg. In fasted dogs, at 10 mg/kg, bioavailability is low (∼ 16%), Cₘₐₓ = 442 ng/mL, Tₘₐₓ = 2.8 h and t_{1/2} = 7.6 h.

The single dose and repeat dose plasma profiles for alpha tocotrienol quinone were simulated using a dose adjusted to achieve a Cₘₐₓ < 10µM and a Cₘᵢₙ >0.5µM. Assuming a daily dose and linear kinetics, for a 70 kg adult the total dose would need to be 379 mg (5.41 mg/kg) to achieve a C₂₄ₕ of 220.5 ng/ml (0.5µM). The dose is adjusted as appropriate, as many patients with methylmalonic aciduria, isovaleric aciduria, or another organic aciduria are children weighing much less than 70 kg.

The starting dose can be estimated based on the United States Food and Drug Administration guidelines titled "Estimating the Maximum Safe Starting Dose in Initial Clinical Trials for Therapeutics in Adult Healthy Volunteers" (July 2005) as well as the International Conference on Harmonisation of Technical Requirements for Registration of Pharmaceuticals for Human Use (ICH) guidelines titled "Guidance on Non-clinical Safety Studies for the Conduct of Human Clinical Trials and Marketing Authorization for Pharmaceuticals" (July 2008). Per ICH guidelines, predicted exposures from the starting dose should not exceed 1/50^{th} the NOAEL (No-Adverse-Observed-Effect-Level) in the more sensitive species on a mg/m² basis. Following a single oral dose of alpha-tocotrienol quinone, the NOAEL was established to be 500 mg/kg for the female rat, i.e. 3,000 mg/m2. This dosage would be equivalent to 81 mg/kg in an adult human. 1/50th of 81 mg/kg is 1.6 mg/kg, i.e. 110 mg for a 70 kg adult, or 16 mg for a 10 kg child. This dose can be administered once, twice, or three times daily.

### Co-administered agents

While the compounds described herein can be administered as the sole active pharmaceutical agent, they can also be used in combination with one or more other agents used in the treatment or suppression of methylmalonic aciduria, isovaleric aciduria, or another organic aciduria. Representative agents useful in combination with the compounds described herein for the treatment or suppression of methylmalonic aciduria, isovaleric aciduria, or another organic aciduria include, but are not limited to, Coenzyme Q, including Coenzyme Q10; idebenone; MitoQ; acetylcarnitine (such as acetyl-L-carnitine or acetyl-DL-carnitine); palmitoylcarnitine (such as palmitoyl-L-carnitine or palmitoyl-DL-carnitine); carnitine (such as L-carnitine or DL-carnitine); quercetine; mangosteen; acai; uridine; N-acetyl cysteine (NAC); polyphenols, such as resveratrol; Vitamin A; Vitamin C; lutein; beta-carotene; lycopene; glutathione; fatty acids, including omega-3 fatty acids such as α-linolenic acid (ALA), eicosapentaenoic acid (EPA), and docosahexaenoic acid (DHA); lipoic acid and lipoic acid derivatives; Vitamin B complex; Vitamin B1 (thiamine); Vitamin B2 (riboflavin); Vitamin B3 (niacin, nicotinamide, or niacinamide); Vitamin B5 (pantothenic acid); Vitamin B6 (pyridoxine or pyridoxamine); Vitamin B7 (biotin); Vitamin B9 (folic acid, also known as Vitamin B11 or Vitamin M); Vitamin B12 (cobalamins, such as cyanocobalamin); inositol; 4-aminobenzoic acid; folinic acid; Vitamin E; other vitamins; and antioxidant compounds.

The co-administered agents can be administered simultaneously with, prior to, or after, administration of the primary compound intended to treat methylmalonic aciduria, isovaleric aciduria, or another organic aciduria.

### Formulations and Routes of Administration

The compounds for use of the invention may be administered in any suitable form that will provide sufficient plasma and/or central nervous system levels of the compounds. The compounds can be administered enterally, orally, parenterally, sublingually, by inhalation (e.g. as mists or sprays), rectally, or topically in unit dosage formulations containing conventional nontoxic pharmaceutically acceptable carriers, excipients, adjuvants, and vehicles as desired. For example, suitable modes of administration include oral, subcutaneous, transdermal, transmucosal, iontophoretic, intravenous, intraarterial, intramuscular, intraperitoneal, intranasal (e.g. via nasal mucosa), subdural, rectal, gastrointestinal, and the like, and directly to a specific or affected organ or tissue. For delivery to the central nervous system, spinal and epidural administration, or administration to cerebral ventricles, can be used. Topical administration may also involve the use of transdermal administration such as transdermal patches or iontophoresis devices. The term parenteral as used herein includes subcutaneous injections, intravenous injection, intraarterial injection, intramuscular injection, intrasternal injection, or infusion techniques. The compounds are mixed with pharmaceutically acceptable carriers, excipients, adjuvants, and vehicles appropriate for the desired route of administration.

In certain embodiments of the invention, especially those embodiments where a formulation is used for injection or other parenteral administration including the routes listed herein, but also including embodiments used for oral, gastric, gastrointestinal, or enteric administration, the formulations and preparations used in the invention are sterile. Sterile pharmaceutical formulations are compounded or manufactured according to pharmaceutical-grade sterilization standards (United States Pharmacopeia Chapters 797, 1072, and 1211; California Business & Professions Code 4127.7; 16 California Code of Regulations 1751, 21 Code of Federal Regulations 211) known to those of skill in the art.

Oral administration is advantageous due to its ease of implementation and patient (or caretaker) compliance. However, patients with methylmalonic aciduria, isovaleric aciduria, or another organic aciduria may have feeding difficulties. Introduction of medicine via feeding tube, feeding syringe, or gastrostomy can be employed in order to accomplish enteric administration. The active compound (and, if present, other co-administered agents) can be enterally administered in sesame oil, or any other pharmaceutically acceptable carrier suitable for formulation for administration via feeding tube, feeding syringe, or gastrostomy.

The term "nutraceutical" has been used to refer to any substance that is a food or a part of a food and provides medical or health benefits, including the prevention and treatment of disease. Hence, compositions falling under the label "nutraceutical" may range from isolated nutrients, dietary supplements and specific diets to genetically engineered designer foods, herbal products, and processed foods such as cereals, soups and beverages. In a more technical sense, the term has been used to refer to a product isolated or purified from foods, and generally sold in medicinal forms not usually associated with food and demonstrated to have a physiological benefit or provide protection against chronic disease. Accordingly, the compounds described for use herein can also be administered as nutraceutical or nutritional formulations, with additives such as nutraceutically or nutritionally acceptable excipients, nutraceutically or nutritionally acceptable carriers, and nutraceutically or nutritionally acceptable vehicles. Such formulations are sometimes called medical foods. Suitable nutraceutically acceptable excipients may include liquid solutions such as a solution comprising one or more vegetable-derived oils, such as sesame oil, and/or one or more animal-derived oils, and/or one or more fish-derived oils.

The compounds described for use herein can be administered in solid form, in liquid form, in aerosol form, or in the form of tablets, pills, powder mixtures, capsules, granules, injectables, creams, solutions, suppositories, enemas, colonic irrigations, emulsions, dispersions, food premixes, and in other suitable forms. The compounds can also be administered in liposome formulations. The compounds can also be administered as prodrugs, where the prodrug undergoes transformation in the treated subject to a form which is therapeutically effective. Additional methods of administration are known in the art.

Injectable preparations, for example, sterile injectable aqueous or oleaginous suspensions, may be formulated according to methods known in the art using suitable dispersing or wetting agents and suspending agents. The sterile injectable preparation may also be a sterile injectable solution or suspension in a nontoxic parenterally acceptable diluent or solvent, for example, as a solution in propylene glycol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono or di-glycerides. In addition, fatty acids such as oleic acid find use in the preparation of injectables.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents. Alternatively, the compound may also be administered in neat form if suitable.

The compounds for use in the present invention can also be administered in the form of liposomes. As is known in the art, liposomes are generally derived from phospholipids or other lipid substances. Liposomes are formed by mono or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound for use in the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., Methods in Cell Biology, Volume XIV, Academic Press, New York, N.W., p. 33 et seq (1976).

The amount of active ingredient that may be combined with the carrier materials to produce a single dosage form can vary depending upon the patient to which the active ingredient is administered and the particular mode of administration. It will be understood, however, that the specific dose level for any particular patient will depend upon a variety of factors including the activity of the specific compound employed; the age, body weight, body area, body mass index (BMI), general health, sex, and diet of the patient; the time of administration and route of administration used; the rate of excretion; drug combination, if any, used; and the progression and severity of the disease in the patient undergoing therapy. The pharmaceutical unit dosage chosen is usually fabricated and administered to provide a defined final concentration of drug in the blood, cerebrospinal fluid, brain tissues, spinal cord tissues, other tissues, other organs, or other targeted region of the body.

Compounds for use in the present invention may be administered in a single daily dose, or the total daily dosage may be administered in divided dosage of two, three or four times daily.

While the compounds for use in the present invention can be administered as the sole active pharmaceutical agent, they can also be used in combination with one or more other agents used in the treatment or suppression of disorders.

When additional active agents are used in combination with the compounds for use in the present invention, the additional active agents may generally be employed in therapeutic amounts as indicated in the Physicians' Desk Reference (PDR) 53rd Edition (1999), or such therapeutically useful amounts as would be known to one of ordinary skill in the art, or as are determined empirically for each patient.

The compounds for use in the present invention and the other therapeutically active agents can be administered at the recommended maximum clinical dosage or at lower doses. Dosage levels of the active compounds in the compositions for use in the present invention may be varied so as to obtain a desired therapeutic response depending on the route of administration, severity of the disease and the response of the patient. When administered in combination with other therapeutic agents, the therapeutic agents can be formulated as separate compositions that are given at the same time or different times, or the therapeutic agents can be given as a single composition.

In one embodiment, the purity of the preparation of the compound, such as a tocotrienol quinone preparation, is measured prior to the addition of any pharmaceutical carriers or excipients, or any additional active agents. For example, if alpha-tocotrienol quinone is prepared according to any of the methods described in International Patent Application No. PCT/US2009/062212 or United States Patent Application No. 12/606,923, the purity of the alpha-tocotrienol quinone is measured on the final product of the method selected, and prior to adding the pharmaceutical carrier(s) or excipient(s) or additional active agent(s). The purity of the desired tocotrienol quinone, or other compound, by weight, can be at least about 20%, at least about 30%, at least about 40%, at least about 50%, at least about 60%, at least about 70%, at least about 75%, at least about 80%, at least about 85%, at least about 90%, at least about 95%, at least about 96%, at least about 97%, at least about 98%, or at least about 99%, prior to the addition of any pharmaceutical carriers or excipients, or any additional active agents. These same numerical purity levels can also be used as by mole fraction, or by any other relative measurement (such as weight/volume).

In another embodiment, the purity of the preparation of the compound, such as a tocotrienol quinone preparation, is measured as a fraction of the desired tocotrienol quinone relative to the total amount of tocotrienol quinones and (if present) tocotrienols in the preparation. For example, a composition containing 100 mg of alpha-tocotrienol quinone, 50 mg of beta-tocotrienol quinone, and 50 mg of gamma-tocotrienol hydroquinone would be described as 50% alpha tocotrienol quinone by weight, irrespective of the amounts of other non-tocotrienol or non-tocotrienol quinone compounds present in the preparation. This measurement of purity would be the same whether measured before or after addition of pharmaceutical carriers or excipients, or before or after addition of any non-tocotrienol/non-tocotrienol quinone active agents. The purity of the desired tocotrienol quinone, or other compound, by weight, can be at least about 20%, at least about 30%, at least about 40%, at least about 50%, at least about 60%, at least about 70%, at least about 75%, at least about 80%, at least about 85%, at least about 90%, at least about 95%, at least about 96%, at least about 97%, at least about 98%, or at least about 99%. These same numerical purity levels can also be used as by mole fraction, or by any other relative measurement (such as weight/volume).

While it is preferable to administer compounds that cross the blood-brain barrier, compounds that do not cross the blood-brain barrier can be delivered to the central nervous system by spinal and epidural administration, or administration to cerebral ventricles. Alpha-tocotrienol quinone crosses the blood-brain barrier in mice.

### Kits

Articles of manufacture and kits containing materials useful for treating methylmalonic aciduria, isovaleric aciduria, or another organic aciduria may be provided. The article of manufacture comprises a container with a label. Suitable containers include, for example, bottles, vials, and test tubes. The containers may be formed from a variety of materials such as glass or plastic. The container holds a compound selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, or a composition comprising an active agent selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone. In one embodiment, the compound is alpha-tocotrienol quinone. In one embodiment, the active agent is alpha-tocotrienol quinone. The label on the container indicates that the composition is used for treating methylmalonic aciduria, isovaleric aciduria, or another organic aciduria, and may also indicate directions for use in treatment.

Kits comprising any one or more of a compound selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, or a composition comprising an active agent selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone may be provided. In some embodiments, the kit comprises the container described above, which holds a compound selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, or a composition comprising an active agent selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone. In other embodiments, the kit comprises the container described above, which holds a compound selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, or a composition comprising an active agent selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, and a second container comprising a vehicle for the compound or composition, such as one or more vegetable-derived oils, such as sesame oil, and/or one or more animal-derived oils, and/or one or more fish-derived oils. In other embodiments, the kit comprises the container described above, which holds a compound selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, or a composition comprising an active agent selected from alpha-tocotrienol quinone, beta-tocotrienol quinone, gamma-tocotrienol quinone, delta-tocotrienol quinone, alpha-tocotrienol hydroquinone, beta-tocotrienol hydroquinone, gamma-tocotrienol hydroquinone, and delta-tocotrienol hydroquinone, where the compound or composition has been pre-mixed with a vehicle for the compound or composition, such as one or more vegetable-derived oils, such as sesame oil, and/or one or more animal-derived oils, and/or one or more fish-derived oils. The kits may further include other materials desirable from a commercial and user standpoint, including other vehicles, buffers, diluents, filters, needles, syringes, and package inserts with instructions for performing any of the methods described herein for treatment of methylmalonic aciduria, isovaleric aciduria, or another organic aciduria.

In other aspects, the kits may be used for any of the methods described herein, including, for example, to treat an individual with methylmalonic aciduria, isovaleric aciduria, or another organic aciduria.

### EXAMPLES

### Example 1

### MMA Cell Line Assay and Initial Screen for Effective Compound

Alpha-Tocotrienol quinone, its redox-silent version (the bis-Boc protected corresponding hydroquinone), and solvent controls were tested for their ability to rescue cells from MMA fibroblasts of the patient diagnosed with MMA, when the cells were stressed by addition of L-buthionine-(S,R)-sulfoximine (BSO), as described in Jauslin et al., Hum. Mol. Genet. 11(24):3055 (2002), Jauslin et al., FASEB J. 17:1972-4 (2003), and International Patent Application WO 2004/003565. EC50 concentrations of test compound and its redox-silent version were determined and compared. The following compound, 2-((6E,10E)-3-hydroxy-3,7,11,15-tetramethylhexadeca-6,10,14-trienyl)-3,5,6-trimethyl-bis(t-butyloxycarbonyl)benzene-1,4-diol, the bis-Boc protected hydroquinone form of alpha-tocotrienol quinone, was used as the "redox-silent" alpha tocotrienol quinone, or αTTQ-RS.

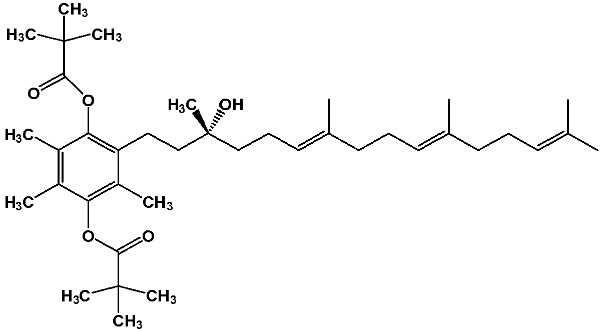

DMEM (Dulbecco's Modified Eagle Medium) -No Glucose, No pyruvate- GIBCO®-Catalog number 11966-025 from Invitrogen. Fetal Calf Serum was obtained from PAA Laboratories. Basic fibroblast growth factor and epidermal growth factor were purchased from PeproTech. Penicillin-streptomycin-glutamine mix, L-buthionine (S,R)-sulfoximine, and insulin from bovine pancreas were purchased from Sigma. Calcein AM was purchased from Molecular Probes. Cell culture medium was made by combining DMEM without glucose, 100 U/mL penicillin, 100 µg/ml streptomycin, 10% FBS, and 5mM Pyruvate. MEM EBS was added to make the volume up to 500 mL. A 10 mM BSO solution was prepared by dissolving 444 mg BSO in 200 mL of medium with subsequent filter-sterilization. This solution was further diluted to 250µM in H₂O. During the course of the experiments, this solution was stored at +4°C. The cells were obtained from the MMA patient and grown in 10 cm tissue culture plates. Every third day, they were split at a 1:3 ratio.

The test samples were supplied in 1.5 mL glass vials. The compounds were diluted with DMSO, ethanol or PBS to result in a 5 mM stock solution. Once dissolved, they were stored at -20 °C.

Test samples were screened according to the following protocol: A culture with MMA fibroblasts was started from a 1 mL vial with approximately 500,000 cells stored in liquid nitrogen. Cells were propagated in 10 cm cell culture dishes by splitting every third day in a ratio of 1:3 until nine plates were available. Once confluent, fibroblasts were harvested. For 54 micro titer plates (96 well-MTP) a total of 14.3 million cells (passage eight) were re-suspended in 480 mL medium, corresponding to 100 µL medium with 3,000 cells/well. The remaining cells were distributed in 10 cm cell culture plates (500,000 cells/plate) for propagation. The plates were incubated overnight at 37°C in an atmosphere with 95% humidity and 5% CO₂ to allow attachment of the cells to the culture plate.

MTP medium (243 µL) was added to a well of the microtiter plate. The test compounds were unfrozen, and 7.5 µL of a 5 mM stock solution was dissolved in the well containing 243 µL medium, resulting in a 150 µM master solution. Serial dilutions from the master solution were made. The period between the single dilution steps was kept as short as possible (generally less than 1 second).

Plates were kept overnight in the cell culture incubator. The next day, 10 µL of a 250µM BSO solution were added to the wells, resulting in a 25 µM final BSO concentration. Thirty hours later, three plates were examined under a phase-contrast microscope to verify that the cells in the 0% control (wells E1-H1) were clearly dead. The medium from all plates was discarded, and the remaining liquid was removed by gently tapping the plate inversed onto a paper towel.

100 µL of PBS containing 1.2 µM Calcein AM were then added to each well. The plates were incubated for 50-70 minutes at room temperature. After that time the PBS was discarded, the plate gently tapped on a paper towel and fluorescence (excitation/emission wavelengths of 485 nm and 525 nm, respectively) was read on a Gemini fluorescence reader. Data was imported into Microsoft Excel (EXCEL is a registered trademark of Microsoft Corporation for a spreadsheet program) and used to calculate the ECso concentration for each compound.

The compounds were tested three times, i.e., the experiment was performed three times, the passage number of the cells increasing by one with every repetition.

The solvents (DMSO, ethanol, PBS) neither had a detrimental effect on the viability of non-BSO treated cells nor did they have a beneficial influence on BSO-treated fibroblasts even at the highest concentration tested (1%). The compounds showed no auto-fluorescence. The viability of non-BSO treated fibroblasts was set as 100%, and the viability of the BSO- and compound-treated cells was calculated as relative to this value.

The results of the cell viability assay for the MMA cells in the presence of alpha-tocotrienol quinone (αTTQ) and redox-silent alpha-tocotrienol quinone (αTTQ-RS) are shown in Figure 1. Alpha-tocotrienol quinone protects the cells with an ED₅₀ of 23 nM, while redox-silent alpha-tocotrienol quinone is ineffective at maintaining cell viability of the fibroblasts cells from the MMA patient.

Hyperlinks disclosed herein to sites on the World-Wide web can be activated and followed by replacing the phrase "World-Wide-Web." with "www."

Although the foregoing invention has been described in some detail by way of illustration and example for purposes of clarity of understanding, it is apparent to those skilled in the art that certain changes and modifications will be practiced. Therefore, the description and examples should not be construed as limiting the scope of the invention.

## Claims

1. A compound selected from the group consisting of tocotrienol quinones and tocotrienol hydroquinones for use in a method of treating or suppressing methylmalonic aciduria, isovaleric aciduria, or another organic aciduria in an individual.

2. The compound of claim 1, for use as defined in said claim, wherein the compound is alpha-tocotrienol quinone.

3. The compound of claim 1, for use as defined in said claim, wherein the compound is alpha-tocotrienol hydroquinone

4. The compound of any one of claims 1 to 3, for use as defined in said claims, for use in a method of treating methylmalonic aciduria.

5. The compound of any one of claims 1 to 3, for use as defined in said claims, for use in a method of treating methylmalonic aciduria, wherein the individual has one or more mutations in the MUT gene.

6. The compound of any one of claims 1 to 3, for use as defined in said claims, for use in a method of treating methylmalonic aciduria, wherein the individual has one or more mutations in the MMAA gene.

7. The compound of any one of claims 1 to 3, for use as defined in said claims, for use in a method of treating methylmalonic aciduria, wherein the individual has one or more mutations in the MMAB gene.

8. The compound of any one of claims 1 to 3, for use as defined in said claims, for use in a method of treating isovaleric aciduria, wherein the individual has at least one mutation in the IVD gene.

9. The compound of any one of claims 1 to 3, for use as defined in said claims, for use in a method of treating an organic aciduria other than methylmalonic aciduria or isovaleric aciduria.

10. The compound of claim 1, for use as defined in said claim, wherein the compound is administered in the form of a pharmaceutical preparation containing from 50 mg to 400 mg of alpha-tocotrienol quinone.

11. The compound of claim 10, for use as defined in said claim, wherein the alpha-tocotrienol quinone comprises at least 80% by weight of the material present in the preparation, excluding the weight of any added pharmaceutical carriers or excipients.

12. The compound of claim 10, for use as defined in said claim, for use in a method of treating methylmalonic aciduria.

13. The compound of claim 10, for use as defined in said claim, for use in a method of treating isovaleric aciduria or another organic aciduria other than methylmalonic aciduria.

14. The compound of claim 10, for use as defined in said claim, (a) for use in a method of treating methylmalonic aciduria, said individual having at least one mutation in the MUT, MMAA, or MMAB genes; or (b) for use in a method of treating isovaleric aciduria, said individual having at least one mutation in the IVD gene.

15. The compound of claim 1, for use as defined in said claim, wherein the individual has one or more symptoms selected from the group consisting of: progressive encephalopathy, dehydration, feeding problems, developmental delays, failure to thrive, lethargy, recurrent yeast infections, seizures, emesis, kidney disease, kidney failure, pancreatitis, coma, brain abnormalities, ventricular dilation, cortical atrophy, periventricular white matter abnormality, thinning of the corpus callosum, subcortical white matter abnormality, cerebellar atrophy, basal ganglionic calcification, and myelination delay.

16. The compound of claim 2, for use as defined in said claim, which is alpha-tocotrienol quinone with the naturally occurring tocotrienol configuration.

17. The compound of claim 1, for use as defined in said claim, wherein the compound is administered in the form of a unit dosage formulation containing from 50mg to 500mg of alpha tocotrienol quinone, wherein the purity of the alpha tocotrienol quinone present in the formulation comprises at least 30% by weight of the tocotrienols and tocotrienol quinones present in the formulation.

18. The compound of claim 1, for use as defined in said claim, wherein the compound is administered in the form of a unit dosage formulation containing from 50mg to 500mg of alpha tocotrienol quinone, wherein the purity of the alpha tocotrienol quinone present in the formulation comprises at least 30% by weight of the material present in the formulation, excluding the weight of any added pharmaceutical carriers of excipients.

19. The compound of any one of claims 1 to 3, for use as defined in said claims, for use in a method of suppressing methylmalonic aciduria.

20. The compound of any one of claims 1 to 3, for use as defined in said claims, for use in a method of suppressing methylmalonic aciduria, wherein the individual has one or more mutations in one or more of the MUT, MMAA and MMAB genes.

## Patentansprüche

1. Verbindung, ausgewählt aus der Gruppe bestehend aus Tocotrienolchinonen und Tocotrienolhydrochinonen zur Verwendung in einem Verfahren des Behandelns oder Unterdrückens von Methylmalonazidurie, Isovalerianazidurie oder einer anderen organischen Azidurie in einem Individuum.

2. Verbindung nach Anspruch 1 zur Verwendung wie in dem Anspruch definiert, wobei die Verbindung alpha-Tocotrienolchinon ist.

3. Verbindung nach Anspruch 1 zur Verwendung wie in dem Anspruch definiert, wobei die Verbindung alpha-Hydrochinon ist.

4. Verbindung nach einem der Ansprüche 1 bis 3 zur Verwendung wie in den Ansprüchen definiert zur Verwendung in einem Verfahren des Behandelns von Methylmalonazidurie.

5. Verbindung nach einem der Ansprüche 1 bis 3 zur Verwendung wie in den Ansprüchen definiert zur Verwendung in einem Verfahren des Behandelns von Methylmalonazidurie, wobei das Individuum eine oder mehrere Mutationen im MUT-Gen hat.

6. Verbindung nach einem der Ansprüche 1 bis 3 zur Verwendung wie in den Ansprüchen definiert zur Verwendung in einem Verfahren des Behandelns von Methylmalonazidurie, wobei das Individuum eine oder mehrere Mutationen im MMAA-Gen hat.

7. Verbindung nach einem der Ansprüche 1 bis 3 zur Verwendung wie in den Ansprüchen definiert zur Verwendung in einem Verfahren des Behandelns von Methylmalonazidurie, wobei das Individuum eine oder mehrere Mutationen im MMAB-Gen hat.

8. Verbindung nach einem der Ansprüche 1 bis 3 zur Verwendung wie in den Ansprüchen definiert zur Verwendung in einem Verfahren des Behandelns von Isovalerianazidurie, wobei das Individuum mindestens eine Mutation im IVD-Gen hat.

9. Verbindung nach einem der Ansprüche 1 bis 3 zur Verwendung wie in den Ansprüchen definiert zur Verwendung in einem Verfahren des Behandelns einer anderen organischen Azidurie als Methylmalonazidurie oder Isovalerianazidurie.

10. Verbindung nach Anspruch 1 zur Verwendung wie in dem Anspruch definiert, wobei die Verbindung in Form eines pharmazeutischen Präparats verabreicht wird, das 50 mg bis 400 mg alpha-Tocotrienolchinon enthält.

11. Verbindung nach Anspruch 10 zur Verwendung wie in dem Anspruch definiert, wobei das alpha-Tocotrienolchinon mindestens 80 Gew.-% des im Präparat vorhandenen Materials umfasst, ausschließlich des Gewichts von hinzugefügten pharmazeutischen Trägern oder Hilfsstoffen.

12. Verbindung nach Anspruch 10 zur Verwendung wie in dem Anspruch definiert zur Verwendung in einem Verfahren des Behandelns von Methylmalonazidurie.

13. Verbindung nach Anspruch 10 zur Verwendung wie in dem Anspruch definiert zur Verwendung in einem Verfahren des Behandelns von Isovalerianazidurie oder einer anderen organischen Azidurie als Methylmalonazidurie.

14. Verbindung nach Anspruch 10 zur Verwendung wie in dem Anspruch definiert (a) zur Verwendung in einem Verfahren des Behandelns von Methylmalonazidurie, wobei das Individuum mindestens eine Mutation im MUT-, MMAA- oder MMAB-Gen hat; oder (b) zur Verwendung in einem Verfahren des Behandelns von Isovalerianazidurie, wobei das Individuum mindestens eine Mutation im IVD-Gen hat.

15. Verbindung nach Anspruch 1 zur Verwendung wie in dem Anspruch definiert, wobei das Individuum eines oder mehrere Symptome hat, ausgewählt aus der Gruppe bestehend aus: progressiver Enzephalopathie, Dehydrierung, Ernährungsproblemen, Entwicklungsverzögerungen, Gedeihstörung, Lethargie, wiederkehrenden Pilzinfektionen, Anfällen, Erbrechen, Nierenerkrankung, Nierenversagen, Pankreatitis, Koma, Hirnanomalien, Ventrikeldilatation, kortikaler Atrophie, Anomalie der periventrikulären weißen Substanz, Ausdünnung des Corpus callosum, Anomalie der subkortikalen weißen Substanz, Kleinhirnatrophie, Basalganglienverkalkung und Myelinisierungsverzögerung.

16. Verbindung nach Anspruch 2 zur Verwendung wie in dem Anspruch definiert, die alpha-Tocotrienolchinon mit der natürlich auftretenden Tocotrienolkonfiguration ist.

17. Verbindung nach Anspruch 1 zur Verwendung wie in dem Anspruch definiert, wobei die Verbindung in Form einer Einheitsdosierungsformulierung verabreicht wird, die 50 mg bis 500 mg alpha-Tocotrienolchinon enthält, wobei die Reinheit des in der Formulierung vorhandenen alpha-Tocotrienolchinons mindestens 30 Gew.-% der in der Formulierung vorhandenen Tocotrienole und Tocotrienolchinone umfasst.

18. Verbindung nach Anspruch 1 zur Verwendung wie in dem Anspruch definiert, wobei die Verbindung in Form einer Einheitsdosierungsformulierung verabreicht wird, die 50 mg bis 500 mg alpha-Tocotrienolchinon enthält, wobei die Reinheit des in der Formulierung vorhandenen alpha-Tocotrienolchinons mindestens 30 Gew.-% des in der Formulierung vorhandenen Materials umfasst, ausschließlich des Gewichts von hinzugefügten pharmazeutischen Trägern und Hilfsstoffen.

19. Verbindung nach einem der Ansprüche 1 bis 3 zur Verwendung wie in den Ansprüchen definiert zur Verwendung in einem Verfahren des Unterdrückens von Methylmalonazidurie.

20. Verbindung nach einem der Ansprüche 1 bis 3 zur Verwendung wie in den Ansprüchen definiert zur Verwendung in einem Verfahren des Unterdrückens von Methylmalonazidurie, wobei das Individuum eine oder mehrere Mutationen im MUT-, MMAA- und MMAB-Gen hat.

## Revendications

1. Composé choisi dans le groupe constitué par tocotriénol quinones et tocotriénol hydroquinones en vue de l'utilisation dans une méthode de traitement ou de suppression d'acidurie méthylmalonique, d'acidurie isovalérique ou d'une autre acidurie organique chez un individu.

2. Composé selon la revendication 1, destiné à une utilisation telle que définie dans ladite revendication, le composé étant : alpha-tocotriénol quinone.

3. Composé selon la revendication 1, destiné à une utilisation telle que définie dans ladite revendication, le composé étant alpha-tocotriénol hydroquinone.

4. Composé selon l'une quelconque des revendications 1 à 3, destiné à une utilisation telle que définie dans lesdites revendications, en vue de l'utilisation dans une méthode de traitement d'acidurie méthylmalonique.

5. Composé selon l'une quelconque des revendications 1 à 3, destiné à une utilisation telle que définie dans lesdites revendications, en vue de l'utilisation dans une méthode de traitement d'acidurie méthylmalonique, l'individu présentant une ou plusieurs mutations dans le gène MUT.

6. Composé selon l'une quelconque des revendications 1 à 3, destiné à une utilisation telle que définie dans lesdites revendications, en vue de l'utilisation dans une méthode de traitement d'acidurie méthylmalonique, l'individu présentant une ou plusieurs mutations dans le gène MMAA.

7. Composé selon l'une quelconque des revendications 1 à 3, destiné à une utilisation telle que définie dans lesdites revendications, en vue de l'utilisation dans une méthode de traitement d'acidurie méthylmalonique, l'individu présentant une ou plusieurs mutations dans le gène MMAB.

8. Composé selon l'une quelconque des revendications 1 à 3, destiné à une utilisation telle que définie dans lesdites revendications, en vue de l'utilisation dans une méthode de traitement d'acidurie isovalérique, l'individu présentant au moins une mutation dans le gène IVD.

9. Composé selon l'une quelconque des revendications 1 à 3, destiné à une utilisation telle que définie dans lesdites revendications, en vue de l'utilisation dans une méthode de traitement d'une acidurie organique autre que l'acidurie méthylmalonique ou l'acidurie isovalérique.

10. Composé selon la revendication 1, destiné à une utilisation telle que définie dans ladite revendication, le composé étant administré sous forme de préparation pharmaceutique contenant de 50 mg à 400 mg d'alpha-tocotriénol quinone.

11. Composé selon la revendication 10, destiné à une utilisation telle que définie dans ladite revendication, dans lequel l'alpha-tocotriénol quinone comprend au moins 80 % en poids du matériau présent dans la préparation, à l'exclusion du poids de quelconques vecteurs ou excipients pharmaceutiques ajoutés.

12. Composé selon la revendication 10, destiné à une utilisation telle que définie dans ladite revendication, en vue de l'utilisation dans une méthode de traitement d'acidurie méthylmalonique.

13. Composé selon la revendication 10, destiné à une utilisation telle que définie dans ladite revendication, en vue de l'utilisation dans une méthode de traitement d'acidurie isovalérique ou d'une autre acidurie organique autre que l'acidurie méthylmalonique.

14. Composé selon la revendication 10, destiné à une utilisation telle que définie dans ladite revendication, (a) en vue de l'utilisation dans une méthode de traitement d'acidurie méthylmalonique, ledit individu présentant au moins une mutation dans les gènes MUT, MMAA, ou MMAB ; ou (b) en vue de l'utilisation dans une méthode de traitement d'acidurie isovalérique, ledit individu présentant au moins une mutation dans le gène IVD.

15. Composé selon la revendication 1, destiné à une utilisation telle que définie dans ladite revendication, dans lequel l'individu présente un ou plusieurs symptômes choisis dans le groupe constitué par : encéphalopathie progressive, déshydratation, problèmes d'alimentation, retards de développement, retard staturo-pondéral, léthargie, lévuroses récidivantes, crises d'épilepsie, vomissement, maladie rénale, insuffisance rénale, pancréatite, coma, anomalies cérébrales, dilatation ventriculaire, atrophie corticale, anomalie de matière blanche périventriculaire, réduction du corps calleux, anomalie de matière blanche sous-corticale, atrophie cérébelleuse, calcification ganglionnaire basale et retard de myélinisation.

16. Composé selon la revendication 2, destiné à une utilisation telle que définie dans ladite revendication, qui est l'alpha-tocotriénol quinone avec la configuration de tocotriénol naturelle.

17. Composé selon la revendication 1, destiné à une utilisation telle que définie dans ladite revendication, le composé étant administré sous forme de formulation de dosage unitaire contenant de 50 mg à 500 mg d'alpha tocotriénol quinone, la pureté de l'alpha tocotriénol quinone présente dans la formulation comprenant au moins 30 % en poids des tocotriénols et tocotriénol quinones présents dans la formulation.

18. Composé selon la revendication 1, destiné à une utilisation telle que définie dans ladite revendication, le composé étant administré sous forme de formulation de dosage unitaire contenant de 50 mg à 500 mg d'alpha tocotriénol quinone, la pureté de l'alpha tocotriénol quinone présente dans la formulation comprenant au moins 30 % en poids du matériau présent dans la formulation, à l'exclusion du poids de quelconques vecteurs ou excipients pharmaceutiques ajoutés.

19. Composé selon l'une quelconque des revendications 1 à 3, destiné à une utilisation telle que définie dans lesdites revendications, en vue de l'utilisation dans une méthode de suppression d'acidurie méthylmalonique.

20. Composé selon l'une quelconque des revendications 1 à 3, destiné à une utilisation telle que définie dans lesdites revendications, en vue de l'utilisation dans une méthode de suppression d'acidurie méthylmalonique, l'individu présentant une ou plusieurs mutations dans les gènes MUT, MMAA et MMAB.
